# EUROPÄISCHE PATENTSCHRIFT

(11) **EP 1 036 322 B1**
(45) Veröffentlichungstag und Bekanntmachung des Hinweises auf die Patenterteilung: **16.04.2003**
(21) Anmeldenummer: 98963501.6
(22) Anmeldetag: 19.11.1998
(51) Int. Cl.: G01N 33/497

(54) **VORRICHTUNG ZUR UNTERSUCHUNG VON ATEMWEGSERKRANKUNGEN UND DIAGNOSTISCHE MITTEL**
DEVICE FOR INVESTIGATING RESPIRATORY TRACT DISEASES AND DIAGNOSTIC AGENTS
DISPOSITIF POUR ETUDIER LES MALADIES DES VOIES RESPIRATOIRES ET AGENTS DIAGNOSTIQUES

(30) Priorität: 03.12.1997 DE 19755471
(43) Veröffentlichungstag der Anmeldung: 20.09.2000
(73) Patentinhaber: UFZ-UMWELTFORSCHUNGSZENTRUM LEIPZIG-HALLE GMBH, 04318 Leipzig (DE)
(72) Erfinder: KRUMBIEGEL, Peter, D-04157 Leipzig (DE); KÖBRICH, Rainer, D-68809 Neulussheim (DE)
(74) Vertreter: Ziebig, Marlene, Dr. Dipl.-Chem.
(86) Internationale Anmeldenummer: EP9807549
(87) Internationale Veröffentlichungsnummer: WO99028743

(56) Entgegenhaltungen:
- WO-A-94/28941
- DE-A- 3 932 784
- DE-A- 19 505 504
- DE-A- 19 528 158
- US-A- 4 803 977

## Beschreibung

Die Erfindung betrifft eine Vorrichtung, die in Verbindung mit einem üblichen Analysensystem zur Diagnose der Stoffwechselleistungen der Atemwegsorgane angewendet werden kann. Mit der Vorrichtung können geeignete Diagnostika in definierten Mengen der Einatmungsluft zudosiert werden und nach einer bestimmten Zeitspanne in der Ausatmungsluft vorhandene Metaboliten des Diagnostikums und/oder das noch vorhandene Diagnostikum selbst aufgefangen werden. Mit üblichen Analysensystemen (z. B. mittels Massenspektrometrie, Gaschromatographie, GC-MS-MS, HPLC-MS, Isotopenanalyse) werden diese ausgeatmeten und aufgefangenen Stoffe identifiziert und mengenmäßig bestimmt, wodurch Rückschlüsse auf den Gesundheitszustand der Atemwegsorgane möglich sind.

Gegenstand der Erfindung sind auch diagnostische Mittel, die zur Bestimmung der Stoffwechselleistung des Atemwegsepithels eingesetzt werden können, besonders vorteilhaft auch in Verbindung mit der erfindungsgemäßen Vorrichtung sowie ein entsprechendes Verfahren zur chemisch-analytischen Bestimmung der Stoffwechselleistung des Bronchienepithels.

Aus dem Stand der Technik ist es seit langem bekannt, flüchtige Substanzen aus der Ausatmungsluft zu sammeln, indem die Luft über einen Sorbenten geleitet und die Substanzen adsorbiert und bestimmt werden. In der Mitte der 70-iger Jahre wurde als hierzu besonders geeigneter Sorbent das sogenannte Tenax, ein auf 2,6-Diphenyl-p-phenylenoxid basierendes Polymer, entwickelt (Krotoszynski et al., J. Chromatogr. Sci. 15, 239-244, 1977; Krotoszynski et al., J. Analyt. Toxicol. 3, 225-234, 1979).
Neuere Studien von Wallace et al., Environmental Health Perspectives, Vol. 104, Supplement 5, S. 861-869, 1996 beschäftigen sich mit der Nützlichkeit der Atemanalyse bei der Bestimmung flüchtiger organischer Substanzen in der Atemluft, nachdem die Probanden bestimmten Chemikalien in ihrer Umwelt ausgesetzt waren (Autoabgase, Tankstellen, Schwimmbäder, Belastung durch Benzol und Styrol beim aktiven Rauchen).

Es sind verschiedene Verfahren und Vorrichtungen zum Sammeln der flüchtigen und auch der nicht-flüchtigen Substanzen aus der Atemluft bekannt geworden.
So beschreibt die WO 91/05255 A1 ein Verfahren, bei dem nicht-flüchtige Biopolymere, wie z. B. Proteine aus der broncho-alveolären Grenzflüssigkeit, in der Atemluft nachgewiesen werden. Hierzu werden von einem Probanden Atemstöße auf ein auf -196°C gekühltes, 1 cm² großes Probenträgerplättchen aufgehaucht. Die Probe wird dann auf dem Cryotisch durch Anlegen eines Ultrahochvakuums gefriergetrocknet und anschließend analysiert.

DE 195 05 504 A1 beschreibt ein Verfahren und eine Vorrichtung zum Sammeln von ausgeatmetem Atemkondensat.

Hier durchströmt die Ausatemluft ein Probensammelrohr und wird in diesem auf eine Minustemperatur unter 0°C abgekühlt, wobei die flüssigen und lösbaren Bestandteile auskondensieren und an der, eine niedrigere Temperatur als das Atemkondensat aufweisenden, Innenwand des Probensammelrohres anfrieren.
Gunther Becher et al. beschreiben in Applied Cardiopulmonary Pathophysiology 5, S. 215-219, 1995 die Bestimmung von Leukotrien B₄(LTB₄) im Atemkondensat, einem Mediator, der im Fall einer Entzündung der Mucosa von den inflammatorischen Zellen in die Atemwege abgegeben wird. Es wird gezeigt, daß die Menge des ausgeatmeten LTB₄ mit dem klinischen Stadium des Bronchialasthmas korreliert. Die Schrift kommt zum Schluß, daß die Sammlung des Atemkondensats und dessen biochemische Analyse zur Diagnose entzündlicher Luftwegserkrankungen gut geeignet ist.

Die LTB4-Bestimmung ist die zur Zeit direkteste Methode, weil sie auf der Messung einer biochemischen endogenen Komponente beruht. Bei dieser Methode besteht jedoch keine Möglichkeit für eine echte Quantifizierung des Krankheitsstadiums durch eine standardisierbare Bezugsgröße. Zum anderen ist eine praktische Anwendung in der Routinediagnostik durch die Notwendigkeit der Anreicherung und Messung sehr geringer Stoffmengen begrenzt, Mengen deren intra- und interindividuelle Schwankungen im Normalbereich größer sein können als z.B. der Einfluß eines Bronchialasthmas.

Das Problem besteht auch darin, daß von den in der Ausatmungsluft vorkommenden Mediatoren wie z. B. Proteinen, Peptiden, Aminosäuren und Phospholipiden in der Mehrzahl noch nicht bekannt ist, wie sie mit dem Krankheitsstadium der Luftwege oder der Lunge korrelieren, so daß eine Diagnose auf dieser Basis noch nicht möglich ist. Auch in WO 91/05255, die ein Verfahren zur Diagnose des Gesundheitszustandes der Lunge und der Atemwege beansprucht, wird lediglich gezeigt, daß nicht-flüchtige Biopolymere in der menschlichen Atemluft analysiert werden können. Welche Rückschlüsse für den Gesundheitszustand der Atemwege und insbesondere für die Stoffwechselleistung des Atemwegsepithels daraus gezogen werden können, geht aus der WO 91/05255 nicht hervor.

Allen bisherigen Lösungen mangelt es daran, daß sie es nicht vermögen, den physiologisch-chemischen Zustand und die metabolische Kapazität des Bronchienepithels über die Atmungsluft zuverlässig zu charakterisieren.

Aufgabe der Erfindung war es deshalb, eine Methode zu entwickeln, um den Gesundheitszustand der Atemwege und insbesondere die Stoffwechselaktivität der Zellen der Atemwegsgrenzfläche sowie den Oberflächenzustand der Bronchialschleimhaut reproduzierbar, zuverlässig, standardisiert und unmittelbar auf chemischanalytischem Wege zu bestimmen, und eine einfache Vorrichtung dafür bereitzustellen.

Erfindungsgemäß wird eine Vorrichtung bereitgestellt, mittels der während der Einatmung eine kleine, genau definierte Menge eines geeigneten Diagnostikums inhaliert wird und während der Ausatmung die zurückflutende Menge dieses Mittels und/oder die seiner Metaboliten durch sukzessive definierte Kühlung und Adsorption aufgefangen und genau bestimmt wird.

Das Diagnosemittel wird vom Bronchienepithel zu einem bestimmten Anteil resorbiert und gegebenenfalls durch eine biochemische Reaktion in definierte Folgeprodukte umgewandelt (metabolisiert). Das Ausmaß der Resorption und der Umwandlung ist vom Gesundheitszustand des Epithelgewebes und damit auch von dessen derzeitiger Stoffwechselleistung abhängig. Der Zustand des Epithels kann somit auf direktem biochemischen Wege bestimmt werden, indem der auf die inhallierte Menge bezogene Anteil des ausgeatmeten Diagnostikums und/oder der entsprechende Anteil seines definierten Metaboliten gemessen wird.

Die erfindungsgemäße Vorrichtung ist gemäß der Ansprüche ausgestaltet. Sie besteht aus einem Strömungskanal 1 mit Mundstück 2 am vorderen Ende, einem Einlaßventil 3, einer Inhalations- oder Injektionseinheit für das Diagnostikum 4, einer Kühlfalle für das Abscheiden der in der Ausatmungsluft vorhandenen löslichen Substanzen 5, die in einem Winkel zum Strömungskanal 1 angeordnet ist, und einem nach der Kühlfalle 5 am Ende des Strömungskanals 1 angeordneten Adsorptionsgefäß 6.

In einer bevorzugten Ausführungsform der Erfindung ist die Längsachse der Kühlfalle in einem Winkel von ca. 45° zum Strömungskanal angeordnet, wodurch ein annähernd gleichmäßiges Durchströmen der Kühlfalle erreicht wird.

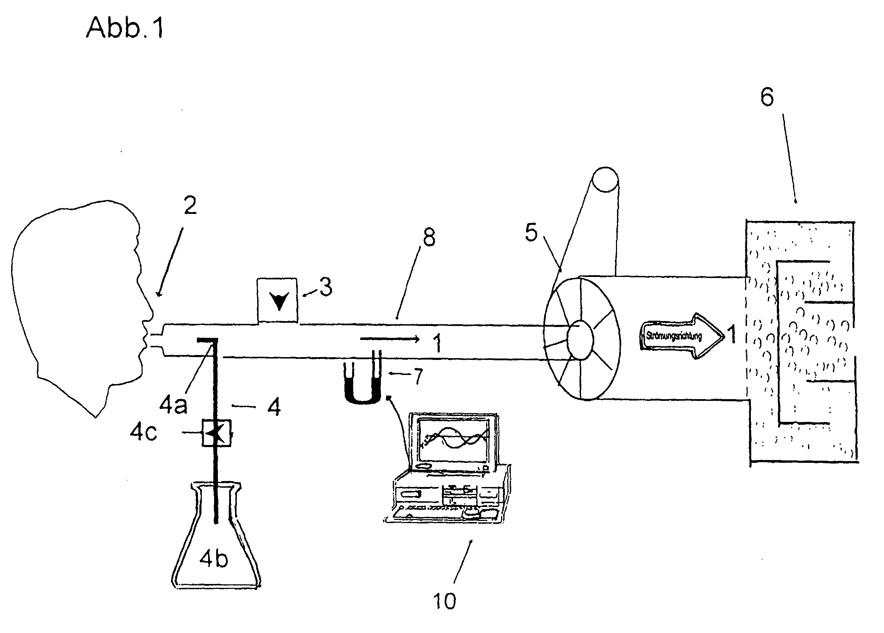

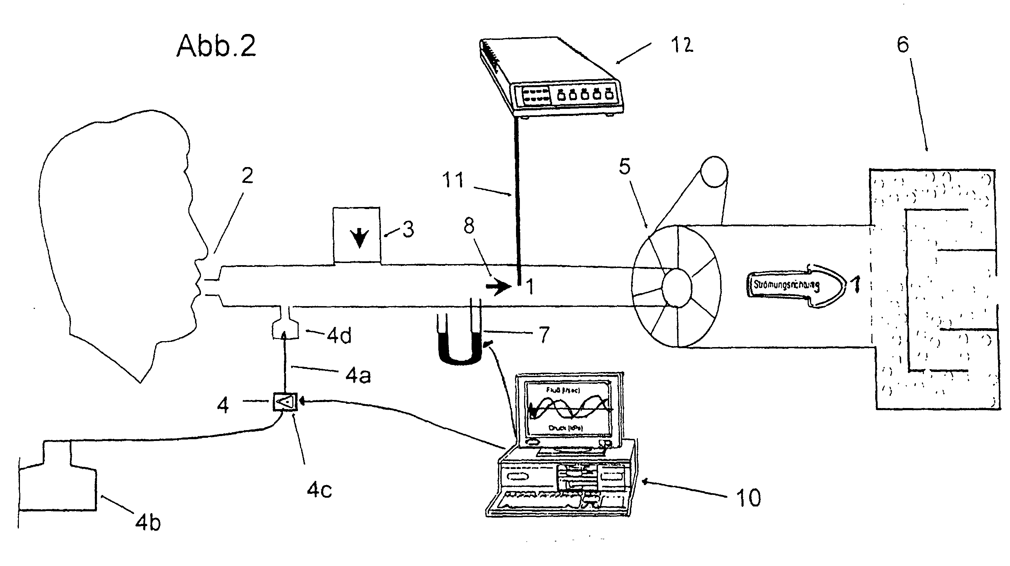

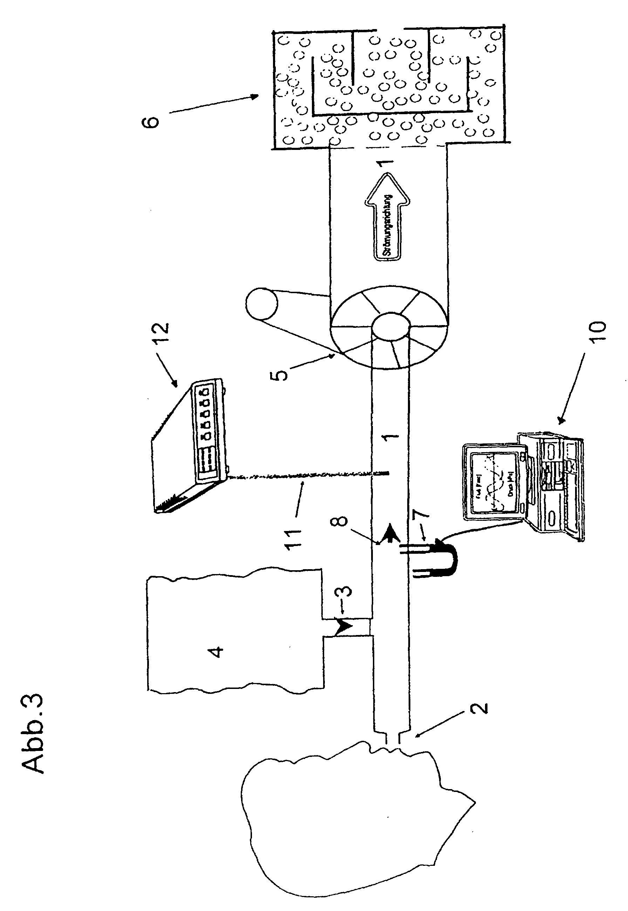

Die Inhalations- oder Injektionseinheit zur Zudosierung des Diagnostikums zur Einatmungsluft 4 kann auf unterschiedliche Weise, aber mit an sich bekannten Geräten oder Geräteteilen ausgestaltet sein. So kann die Zudosierung z.B. mit einer Dosierpumpe oder Kolbenspritze erfolgen oder durch einfaches Ansaugen des Diagnostikums in einer entsprechenden Verdünnung (vgl. Abb. 1). Auch eine vorherige Vernebelung oder Zerstäubung des Diagnostikums ist möglich. Bevorzugt kann z.B. eine PC-gsteuerte Aerosolherstellung unter Verwendung eines Druckluft-, Düsen- oder Ultraschallverneblers oder Zentrifugalzerstäubers zum Einsatz kommen (vgl. Abb. 2). In diesen Fällen wird über das Einlaßventil 3 die Frischluft angesaugt. Es stellt für den Fachmann jedoch kein Problem dar, auch andere geeignete Inhalations- oder Injektionseinheiten bereitzustellen. So kann z.B. auch in einem Reservoirbeutel ein vorbereitetes Luft-Diagnostikum-Gemisch bereitgestellt und über das Einlaßventil 3 eingeatmet oder zudosiert werden (vgl. Abb. 3).

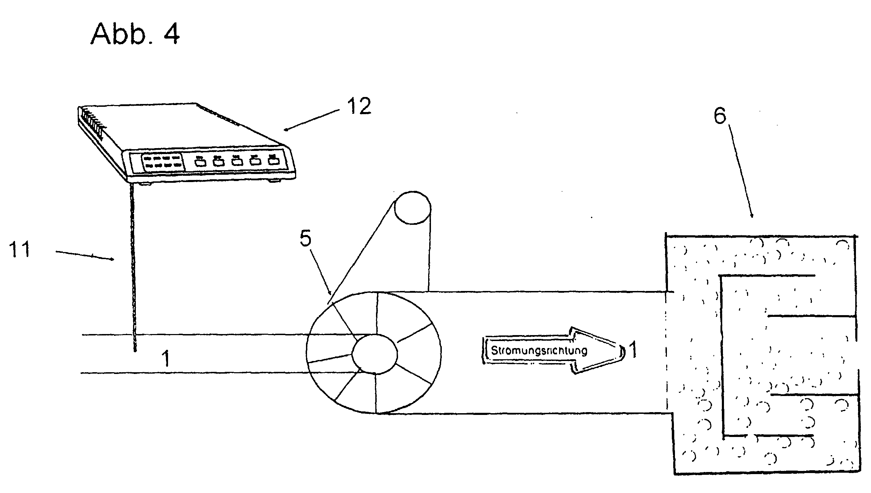

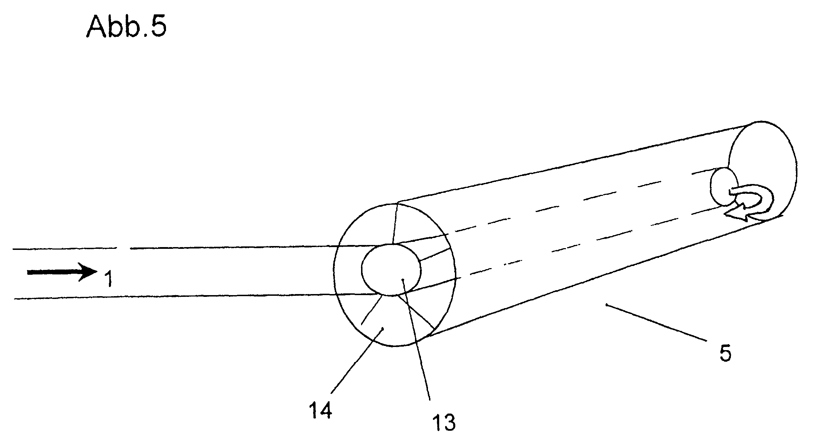

Gemäß der Vorrichtung der Erfindung durchströmt die zu analysierende Ausatmungsluft zum Sammeln der in ihr vorhandenen Metaboliten des Diagnostikums und/oder des noch vorhandenen Diagnostikums selbst zunächst die Kühlfalle 5, in der das in der Ausatmungsluft vorhandene Wasser zusammen mit den vorhandenen nicht gasförmigen Substanzen auskondensiert wird. Die Kühlfalle 5 kann z.B., um eine optimale Kühlleistung zu erreichen, eine Umkehrkühlfalle, wie in Abb. 5 dargestellt, sein. Aber auch jede andere Ausgestaltung ist möglich, wichtig ist lediglich, daß der zur Kühlleisung einstellbare Temperaturbereich von mindestens -5°C bis -25°C reicht. Dies kann auf sehr einfache Weise durch ein Kühlmantelrohr erreicht werden, durch welches Wasser mit einem Kühlmittelzusatzt geleitet wird. Bei Einsatz einer Umkehrkühlfalle gemäß Abb. 5 kann diese an ein Kühlaggregat angeschlossen werden. Die Kühlfalle 5 kann aus der Vorrichtung herausnehmbar angeordnet sein, so daß das Atemkondensat in gefrorenem Zustand gesammelt werden kann. Es ist aber auch möglich, bei einer computergesteuerten Ausgestaltung der Vorrichtung die Kühlfalle 5 nach Abschluß des Sammelns des Exhalates zu erwärmen und das die zu bestimmenden Substanzen enthaltende Wasser direkt in die Analyseeinheit 12 abzusaugen (vgl. Abb. 4). Erfindungsgemäß kann die Vorrichtung auch mehrere hintereinandergeschaltete Kühlfallen enthalten.

Nach der Kühlfalle 5 passieren die gasförmigen Bestandteile und verbleibenden Reste des Exhalats ein Adsorptionsgefäß 6. Das Adsorptionsgefäß 6 enthält einen zum Sammeln von flüchtigen Substanzen in der Ausatmungsluft üblichen Sorbenten, z.B. ein organisches Polymer oder Aktivkohle. Grundsätzlich schlägt sich das Atemkondensat jedoch an der Innenwand der Kühlfalle 5 nieder.

Wie für den Fachmann unschwer zu erkennen ist, kann die Vorrichtung in zwei verschiedenen Weisen betrieben werden, entweder mittels mechanischer Ventile oder durch eine computergesteuerte Ventilschaltung. Die passive Strömungssteuerung mittels eines mechanischen Ventils 3 ermöglicht eine rein mechanische Trennung des inspiratorischen und exspiratorischen Strömungsweges. Das leichtgängige Ventil 3 öffnet sich schon bei dem geringsten Unterdruck (Inspiration) innerhalb des Mundstückes, so daß das inspirerte Volumen immer über diesen Einlaß zuströmt. Sobald der Unterdruck abklingt oder es zu einem Überdruck am Mundstück kommt (Atemstillstand bzw. Ausatmungsluft), verschließt sich das Ventil selbständig. Die Kühl- und Adsorptionsstrecke zeichnet sich durch einen systemeigenen Widerstand aus, der ein Zuströmen während der Inspiration ausschließt. Bei geringen Strömungsdifferenzen (z. B. bei Messungen an Kindern) wird ein zusätzliches passives Expirationsventil eingesetzt. Bei der Ausatmung gibt es für die Ausatemluft nur den Strömungsweg über die Kühl- und Adsorptionsstrecke.

Alternativ zu der passiven Variante gibt es bei Verwendung von PC- gesteuerten Ventilen die Möglichkeit, exspirationsvolumen- und/oder strömungsgeschwindigkeitsabhängig die Öffnung der Kühl- und Adsorptionsstrecke zu realisieren.

Das gesammelte Atemkondensat kann also direkt, d.h. online, durch Absaugung der ausgefrorenen und wieder aufgetauten Substanzen der Analyseneinheit 12, z. B. einer üblichen Kombination aus Chromatograph und Massenspektrometer, zugeführt werden. Bei einer herausnehmbar gestalteten Kühlfalle 5 wird das Atemkondensat manuell durch Auftauen von der Innenwand der Kühlfalle gelöst und der Analyse zugeführt oder bis zur Messung im tiefgefrorenen Zustand aufbewahrt.
In einer besonders bevorzugten Ausführungsform der Erfindung wird der Patient zwischen zwei Untersuchungen einer in der Diagnose von Atemwegserkrankungen bekannten Kaltluftprovokation unterworfen, wodurch eine Differenzmessung für Art und Menge der ausgeatmeten chemischen Substanzen vor und nach einer Schleimhautreizung stattfinden kann. Zur Kaltluftprovokation wird vorzugsweise eine an sich bekannte Luftkühlungsvorrichtung dem Einlaßventil 3 vorgeschaltet, z.B. das Kaltluftprovokationsgerät RHES der Fa. Jäger, Würzburg. In der erfindungsgemäßen Vorrichtung kann diese Luftkühlungsvorrichtung auch zur Kühlung des Innenrohres der Kühlfalle 5 eingesetzt werden. Durch diese Differenzmessung wird eine wesentliche Erhöhung der Zuverlässigkeit der Analysenergebnisse erreicht.

Die erfindungsgemäße Analyse des im Exhalat noch vorhandenen Diagnostikums oder dessen Metaboliten erfolgt mit üblichen Analysemethoden in Abhängigkeit vom eingesetzten Diagnostikum, vorzugsweise durch massenspektrometrische und chromatographische Untersuchungen.

Um eine gute Verträglichkeit des Diagnostikums zu gewährleisten, werden dafür bevorzugt Zubereitugen mit körpereigenen und körperverwandten Stoffen eingesetzt, vorzugsweise z.B. Aminosäuren oder höhere Alkohole in großer Verdünnung, wie sie in den Ansprüchen näher gekennzeichnet sind. Diese Verbindungen haben sich als geeignete Diagnostika für das vorliegende Untersuchungsverfahren erwiesen. Die eingesetzten diagnostischen Mittel umfassen gegebenenfalls pharmazeutisch übliche Zusatzstoffe.

Um eine hohe Spezifität der Diagnose zu gewährleisten und die Sensitivität der Bestimmung zu optimieren, werden diese Stoffe in einer stabilisotop (d.h.: nichtradioaktiv) markierten Form verabreicht. Nur auf diese Weise kann das wieder ausgeatmete Diagnostikum oder sein spezifisches Umwandlungsprodukt als Teil des eingeatmeten Diagnosemittels identifiziert werden.

Somit ist der in einer bestimmten Zeit wiedergefundene Anteil des stabilisotop markierten Diagnosemittels (oder seines definierten Folgeprodukts) ein zuverlässiges Maß für die aktuelle Resorptions- und Stoffwechselleistung des Epithels.

Gegenstand der Erfindung ist auch die neue Verwendung von höheren, pharmazeutisch verträglichen Alkoholen oder Aminosäuren in stabilisotop markierter Form zur Diagnose von Atemwegserkrankungen sowie ein Verfahren zur chemisch-analytischen Bestimmung der Stoffwechselleistung des Bronchienepithels mittels der erfindungsgemäßen Vorrichtung. Die Probanden inhalieren Diagnostika, die in den Atemwegsorganen Metabolite bilden können, anschließend wird die Ausatmungsluft gesammelt und die Metaboliten und/oder die verbliebene Menge des Diagnostikums werden bestimmt, nachdem sie durch Ausfrieren und Adsorption wiedergewonnen wurden.

In einer bevorzugten Ausführungsvariante inhaliert ein Proband als Diagnostikum einen stabilisotop markierten höheren, pharmazeutisch verträglichen Alkohol. Nach einer Einwirkdauer von ca. 1 bis 3 Stunden, vorzgsweise 1 bis 2 Stunden, wird die Ausatmungsluft gesammelt und das ausgeatmete Diagnostikum bzw. dessen Metaboliten werden ausgefroren. Anschließend wird die Menge und/oder der Isotop-Gehalt der Substanzen bestimmt und mit dem Isotop-Basiswert in der Atemluftnullprobe des Probanden verglichen.

Bevorzugte Diagnostika sind höhere Alkohole mit mindestens 8 C-Atomen vorzugsweise Hexadecanol-1, welches bevorzugt 13C-markiert ist.

In einer weiteren bevorzugten Ausführungsvariante werden als Diagnostika stabilisotop ¹⁵N-markierte Aminosäuren eingesetzt. Die Ausatmungsluft wird vom ersten Ausatmungsvorgang über einen Zeitraum von mindestens 30 Minuten gesammelt und nach an sich bekannten Methoden gezielt auf gasförmige Metaboliten des Diagnostikums oder N-markierte nitrose Gase analysiert. Bevorzugtes Diagnostikum ist als ¹⁵N-markierte Aminosäure die Aminosäure L-Arginin.

In einer weiteren bevorzugten Variante wird nach der Sammlung der Ausatmungsluft eine Kaltluftprovokation des Probanden durchgeführt und das Diagnostikum noch einmal zum Inhalieren gegeben. Die Bestimmung der Ausatmungsluft wird wiederholt und diese Meßwerte werden zu den Meßwerten vor der Provokation ins Verhältnis gesetzt, wodurch die Empfindlichkeit für die Bronchienoberfläche bestimmt sowie auch der Schweregrad einer Entzündung ermittelt werden kann.

Es zeigen die Abbildungen:
- Abb. 1: Erfindungsgemäße Vorrichtung mit Frischluftventil 3 und einfacher Inhalations- oder Injektionseinheit 4
- Abb 2: Erfindungsgemäße Vorrichtung mit Frischluftventil 3 und Aerosolinhalations- oder injektionseinheit 4
- Abb. 3: Erfindungsgemäße Vorrichtung mit antistatischem Reservoirbeutel
- Abb. 4: Ausschnit aus der erfindungsgemäßen Vorrichtung mit Absaugevorrichtung 11, Analyseeinheit 12, Kühlfalle 5 und Adsorptionsstrecke 6
- Abb. 5: Umkehrkühlfalle 5

### Ausführungsbeispiele

### Beispiel 1

Zu 5 L Luft in einem geschlossenen Behälter wird mit dem stabilen Isotop ¹³C markiertes Hexadecanol-1 (mehr als 95 Atom-% ¹³C in der CH₂OH-Gruppe enthaltend) in definierten Mengen zwischen 1 und 100 mg zugemischt. [1-¹³C]Hexadecanol dient erfindungsgemäß als Diagnostikum für die Messung der Durchlässigkeit des Bronchienepithels. Vor der Applikation der ¹³C-Hexadecanol-1-Zubereitung wird eine Atemluftprobe des zu untersuchenden Individuums aufgefangen ("Nullprobe"), um darin den aktuellen natürlichen ¹³C-Basiswert des ausgeatmeten Kohlendioxids dieser Person zu messen. Der Basiswert wird auf den sogenannten PDB-Standardwert bezogen, der 1,1112328 Atom-% ¹³C beträgt. Von Individuum zu Individuum weicht dieser Wert ab der 2. Stelle hinter dem Komma vom Standardwert ab, und diese Abweichung kann sehr genau gemessen werden. Dazu dient in an sich bekannter Weise ein Massenspektrometer oder ein spezielles ¹³C-Atemgasmeßgerät (z.B. das Gerät FANci2 der Fa. Fischer Analysen Instrumente Leipzig).

Anschließend wird mit 10 Atemzügen das vorbereitete Luft-Hexadecanol-Gemisch mit Hilfe der erfindungsgemäßen Anordnung der Abb. 3 komplett eingeatmet. Nach 1 und nach 2 Stunden Wartezeit wird die Ausatmungsluft von je 50 Ausatmungen mit der erfindungsgemäßen Anordnung gesammelt. Das wieder ausgeatmete [1-¹³C]Hexadecanol und das Kohlendioxid wird durch fraktioniertes Ausfrieren separiert. Die Menge und der ¹³C-Gehalt der beiden Substanzen werden bestimmt.

Der ¹³C-Wert des Kohlendioxids der Ausatmungsluft weicht nun in charakteristischer Weise von dem der Nullprobe ab: Der Wert ist umso stärker erhöht (z.B. von 1,11127 auf 1,2467...Atom-% ¹³C) je mehr [1-¹³C] Hexadecanol das Bronchienepithel durchdrungen hat; denn nur nach dem Überwinden dieser Schranke kann das Hexadecanol in den Blutkreislauf gelangen und in der Leber zu Kohlendioxid abgebaut werden. Die Menge des aus dem [1-¹³C]Hexadecanol stammenden und im CO₂ der Ausatmungsluft wiedergefundenen Isotops ¹³C ist demzufolge ein zuverlässiges Maß für die Durchlässigkeit des Bronchienepithels.

### Beispiel 2

Die Apparatur wird wie im Beispiel 1 genutzt. Zusätzlich wird aber nach der Sammlung der Ausatmungsluft eine Kaltluftprovokation durchgeführt, indem eine in der erfindungsgemäßen Anordnung dem Ventil 3 vorschaltbare an sich bekannte Luftkühlungsvorrichtung RHES der Fa. Jäger, Würzburg genutzt wird. 20 Minuten nach der Kaltluftprovokation werden die im Beispiel 1 erläuterten Schritte des Zumischens, Einatmens, Ausatmens und Messens noch einmal in derselben Weise wiederholt. Die nach der Kaltluftprovokation gemessenen ¹³C-Kohlendioxidwerte werden zu den Meßwerten vor der Provokation ins Verhältnis gesetzt. Dadurch wird für die Empfindlichkeit der Bronchienoberfläche eine intraindividuell geeichte und somit interindividuell vergleichbare Maßzahl erhalten.

### Beispiel 3

Dem Vorratsluftbehälter werden zwischen 1 und 100 mg einer in physiologischer Kochsalzlösung gelösten ¹⁵N-markierten Aminosäure, vorzugsweise L-[guanino-¹⁵N₂] Arginin, zugemischt. Dieses handelsübliche ¹⁵N-markierte Arginin enthält z.B. 95 Atom-% ¹⁵N in der Guaninogruppe. Mit bis zu 20 Atemzügen wird das vorbereitete Luft-Aerosol-Gemisch mit der erfindungsgemäßen Anordnung der Abb. 3 komplett eingeatmet, wobei die Ausatmungsluft erfindungsgemäß über das Ventil 8 vom ersten Ausatmungsvorgang an über die Kühlfallen- und Adsoberstrecke geführt wird, und zwar über eine Gesamtzeit von 60 Minuten, wobei nach 30 Minuten die Kühlfalle und der Adsorber gewechselt werden. Nach dieser Zeit wird der Inhalt der Kühlfallen und der Adsorberstrecken der GC-MS-Analyse zugeführt. Die mit der Ausatmungsfeuchtigkeit zusammen ausgefrorenen Substanzen und die adsorbierten Substanzen werden gezielt auf die gasförmigen Metaboliten des [¹⁵N₂]Arginins, d.h., auf [¹⁵N]Ammoniak und ¹⁵N-markierte nitrose Gase untersucht. Wird praktisch kein ¹⁵N-markiertes Ammoniak oder Stickstoffoxid gefunden, so war die Stoffwechselaktivität des Bronchienepithels normal, d.h. relativ gering. Im Streß - bzw. Entzüngungszustand hingegen ist die Stoffwechselaktivität stark erhöht. Dies äußert sich in einer vergleichsweise großen Menge an ¹⁵N-markierten gasförmigen Substanzen im Ausatmungskondensat, die nur von dem ¹⁵N-markierten Arginin stammen können. Die Menge des im Kondensat wiedergefundenen Isotops ¹⁵N ist demzufolge ein zuverlässiges Maß für die Stoffwechselaktivität und für den Entzündungsgrad des Bronchienepithels. Zusätzlich kann auch der Urin auf ¹⁵N-haltige Metaboliten untersucht werden.

### Beispiel 4

Die Apparatur wird wie im Beispiel 3 genutzt. Nach Beendigung der einstündigen Sammlung von Ausatmungskondensat und ¹⁵N-markierten Gasen erfolgt eine Kaltluftprovokation in an sich bekannter Weise wie in Beispiel 2. Dadurch kann man die Empfindlichkeit des Bronchienepithels gegenüber einer Kaltluftprovokation erstmals biochemisch messen - und zwar nichtinvasiv und in vivo in Form eines reduzierten oder erhöhten Aminosäure-Metabolismus. Aus dem Verhältnis der ¹⁵N-Mengen der ausgeatmeten Gase aus dem eingeatmeten Arginin vor und nach der Kaltluftprovokation kann auch der Schweregrad der Entzündung ermittelt werden.

### Bezugszeichenliste

### Abb. 1

- 1: Strömungskanal
- 2: Mundstück
- 3: Einlaßventil für Frischluft
- 4: Inhalations- oder Injektionseinheit
- 4a: Zuleitung
- 4b: Gefäß für Diagriostikum
- 4c: Ventil
- 5: Kühlfalle
- 6: Absorptionsgefäß
- 7: Strömungsmesser
- 8: Ventil
- 10: Computersteuerung

### Abb. 2

- 1: Strömungskanal
- 2: Mundstück
- 3: Einlaßventil für Frischluft
- 4: Inhalations- oder Injektionseinheit
- 4a: Zuleitung
- 4b: Gefäß für Diagnostikum
- 4c: Ventil
- 4d: Vernebelungsvorrichtung für das Diagnostikum
- 5: Kühlfalle
- 6: Absorptionsgefäß
- 7: Strömungsmesser
- 8: Ventil
- 10: Computersteuerung
- 11: Absaugevorrichtung
- 12: Analyseneinheit

### Abb. 3

- 1: Strömungskanal
- 2: Mundstück
- 3: Einlaßventil für Luft-Diagnostikum-Gemisch
- 4: Reservoirbeutel mit Luft-Diagnostikum-Gemisch
- 5: Kühlfalle
- 6: Absorptionsgefäß
- 7: Strömungsmesser
- 8: Ventil
- 10: Computersteuerung
- 11: Absaugevorrichtung
- 12: Analyseneinheit

### Abb. 4

- 1: Strömungskanal
- 5: Kühlfalle
- 6: Absorptionsgefäß
- 11: Absaugevorrichtung
- 12: Analyseneinheit

### Abb. 5

- 1: Strömungskanal
- 5: Kühlfalle
- 13: Innenrohr
- 14: Außenkörper mit Kühlrippen

## Patentansprüche

1. Vorrichtung zur Untersuchung von Atemwegserkrankungen durch Zudosierung von Diagnostika zur Atmungsluft, die in den Atemwegsorganen Metabolite bilden können, und Sammeln der in der Ausatmungsluft vorhandenen Substanzen
bestehend aus
einem Strömungskanal (1) mit Mundstück (2) am vorderen Ende, einem Einlaßventil (3), einer Inhalations- oder Injektionseinheit für das Diagnostikum (4), einer Kühlfalle für das Abscheiden der in der Ausatmungsluft vorhandenen Substanzen (5), die in einem Winkel zum Strömungskanal (1) angeordnet ist, und einem nach der Kühlfalle (5) am Ende des Strömungskanals (1) angeordneten Adsorptionsgefäß (6).

2. Vorrichtung nach Anspruch 1,
**dadurch gekennzeichnet, daß**
die Inhalations- oder Injektionseinheit (4) eine Zuleitung (4a), ein Gefäß für das Diagnostikum (4b) und ein Ventil (4c) umfaßt und das Einlaßventil (3) ein Einlaßventil für Frischluft ist.

3. Vorrichtung nach Anspruch 1 oder 2,
**dadurch gekennzeichnet, daß**
die Inhalations- oder Injektionseinheit (4) eine Vernebelungsvorrichtung (4d) beinhaltet und das Einlaßventil (3) ein Einlaßventil für Frischluft ist.

4. Vorrichtung nach Anspruch 1,
**dadurch gekennzeichnet, daß**
die Inhalations- oder Injektionseinheit (4) ein Reservoirbeutel mit einem vorbereiteten Luft-Diagnostikum-Gemisch ist, der über das Einlaßventil (3) mit dem Strömungskanal (1) verbunden ist.

5. Vorrichtung nach einem der Ansprüche 1 bis 3,
**dadurch gekennzeichnet, daß**
vor das Einlaßventil (3) ein Kaltluftprovokationsgerät geschaltet ist.

6. Vorrichtung nach einem der Ansprüche 1 bis 5,
**dadurch gekennzeichnet, daß**
sie computergesteuert ausgestaltet ist.

7. Vorrichtung nach einem der Ansprüche 1 bis 6,
**dadurch gekennzeichnet, daß** im Strömungskanal (1), ein Strömungsmesser (7) angeordnet ist.

8. Vorrichtung nach Anspruch 6 oder 7,
**dadurch gekennzeichnet, daß**
im Strömungskanal (1) vor der Kühlfalle (5) eine Absaugevorrichtung (11) angeordnet ist.

9. Vorrichtung nach Anspruch 8,
**dadurch gekennzeichnet, daß**
die Absaugevorrichtung (11) mit einer Analyseneinheit (12) verbunden ist.

10. Diagnostisches Mittel zur Inhalation zur Bestimmung des Gesundheitszustandes der Atemwegsorgane anhand der Ausatmungsluft umfassend stabilisotop markierte höhere, pharmazeutisch verträgliche Alkohole und gegebenenfalls pharmazeutisch übliche Zusatzstoffe.

11. Diagnostisches Mittel nach Anspruch 10,
**dadurch gekennzeichnet, daß**
die höheren Alkohole mindestens C₈-Alkohole sind.

12. Diagnostisches Mittel nach Anspruch 10 oder 11,
**dadurch gekennzeichnet, daß**
die höheren Alkohole ¹³C-markiert sind.

13. Diagnostisches Mittel nach einem der Ansprüche 10-12,
**dadurch gekennzeichnet, daß**
der stabilisotop markierte höhere Alkohol ¹³Cmarkiertes Hexadecanol-1 ist.

14. Diagnostisches Mittel zur Inhalation zur Bestimmung des Gesundheitszustandes der Atemwegsorgane anhand der Ausatmungsluft umfassend stabilisotop markierte Aminosäuren und gegebenenfalls pharmazeutisch verträgliche Zusatzstoffe, wobei die Aminosäuren ¹⁵N- markiert sind.

15. Diagnostisches Mittel nach Anspruch 14,
**dadurch gekennzeichnet, daß**
die Aminosäure L-Arginin ist.

16. Verwendung von stabilisotop markierten höheren, pharmazeutisch verträglichen Alkoholen oder stabilisotop markierten Aminosäuren zur Bestimmung des Gesundheitszustandes der Atemwegsorgane anhand der Ausatmungsluft, wobei diese Verbindungen inhaliert werden.

17. Verfahren zur Bestimmung der Stoffwechselleistung des Bronchienepithels
**dadurch gekennzeichnet, daß**
mittels der Vorrichtung nach einem der Ansprüche 1 bis 9 der Atmungsluft eines Probanden Diagnostika, die in den Atemwegsorganen Metabolite bilden können, zudosiert werden, anschließend die in der Ausatmungsluft vorhandenen Substanzen gesammelt werden und die Metabolite und/oder die verbliebene Menge des Diagnostikums analysiert werden.

## Claims

1. A device for examining respiratory tract diseases by dosing diagnostic agents into the respiratory air, which are capable of forming metabolites within the respiratory organs, and collecting the substances present in the expired air,
said device being comprised of
a flow channel (1) with a mouthpiece (2) on the front end, an inlet valve (3), an inhalation or injection unit (4) for said diagnostic agent, a cold trap (5) to precipitate the substances present in the expired air, said cold trap being arranged at an angle relative to the flow channel (1), and an adsorption vessel (6) arranged downstream of cold trap (5) at the end of flow channel (1).

2. The device according to claim 1,
**characterized in that**
the inhalation or injection unit (4) comprises a supply line (4a), a vessel (4b) for the diagnostic agent, and a valve (4c), and that the inlet valve (3) is an inlet valve for fresh air.

3. The device according to claim 1 or 2,
**characterized in that**
the inhalation or injection unit (4) includes a nebulizer (4d), and that the inlet valve (3) is an inlet valve for fresh air.

4. The device according to claim 1,
**characterized in that**
the inhalation or injection unit (4) is a reservoir bag including a previously prepared air/diagnostic agent mixture, which bag communicates with the flow channel (1) via the inlet valve (3).

5. The device according to any of claims 1 to 3,
**characterized in that**
a cold-air provocation apparatus is arranged upstream of inlet valve (3).

6. The device according to any of claims 1 to 5,
**characterized in that**
the device is designed so as to be computer-controlled.

7. The device according to any of claims 1 to 6,
**characterized in that**
a flow meter (7) is arranged inside the flow channel (1).

8. The device according to claim 6 or 7,
**characterized in that**
a suction device (11) is arranged inside the flow channel (1) upstream of cold trap (5).

9. The device according to claim 8,
**characterized in that**
the suction device (11) is connected to an analytical unit.

10. A diagnostic agent to be inhaled, used to determine the state of health of the respiratory organs using the expired air, said agent comprising stable isotope-labelled, pharmaceutically tolerable higher alcohols and optionally pharmaceutically conventional additives.

11. The diagnostic agent according to claim 10,
**characterized in that**
the higher alcohols are at least C₈ alcohols.

12. The diagnostic agent according to claim 10 or 11,
**characterized in that**
the higher alcohols are ¹³C-labelled.

13. The diagnostic agent according to any of claims 10 to 12,
**characterized in that**
the stable isotope-labelled higher alcohol is ¹³C-labelled hexadecan-1-ol.

14. A diagnostic agent to be inhaled, used to determine the state of health of the respiratory organs using the expired air, said agent comprising amino acids labelled with a stable isotope, and optionally pharmaceutically tolerable adjuvants, said amino acids being ¹⁵N-labelled.

15. The diagnostic agent according to claim 14,
**characterized in that**
the amino acid is L-arginine.

16. Use of pharmaceutically tolerable higher alcohols labelled with a stable isotope or of amino acids labelled with a stable isotope in determining the state of health of the respiratory organs using the expired air, wherein said compounds are inhaled.

17. A method of determining the metabolic performance of the bronchial epithelium,
**characterized in that**
diagnostic agents capable of forming metabolites in the respiratory organs are dosed into the respiratory air of a subject using the device according to any of claims 1 to 9, the substances present in the expired air are collected subsequently, and the metabolites and/or remaining amount of diagnostic agent are subjected to analysis.

## Revendications

1. Dispositif de recherche de maladies des voies respiratoire par ajout dosé dans l'air inhalé d'agents de diagnostic pouvant former des métabolites dans les organes des voies respiratoires, et recueil des substances contenues dans l'air expiré,
constitué
d'un canal d'écoulement (1) comportant un bec de sortie (2) à l'extrémité antérieure, d'une soupape d'admission (3), d'une unité d'inhalation ou d'injection (4) de l'agent de diagnostic, d'un piège à refroidissement (5) pour séparer les substances présentes dans l'air expiré, qui est disposé à un angle par rapport au canal d'écoulement (1), et d'un récipient d'adsorption (6) disposé après le piège à refroidissement (5) à l'extrémité du canal d'écoulement (1).

2. Dispositif selon la revendication 1,
**caractérisé en ce que**
l'unité d'inhalation ou d'injection (4) comporte une admission (4a), un récipient (4b) pour l'agent de diagnostic et une soupape (4c), et que la soupape d'admission (3) est une soupape d'admission d'air frais.

3. Dispositif selon la revendication 1 ou 2,
**caractérisé en ce que**
l'unité d'inhalation ou d'injection (4) comporte un dispositif de nébulisation (4d), et que la soupape d'admission (3) est une soupape d'admission d'air frais.

4. Dispositif selon la revendication 1,
**caractérisé en ce que**
l'unité d'inhalation ou d'injection (4) est un sachet réservoir contenant un mélange air-agent de diagnostic préparé, qui est relié au canal d'écoulement (1) par la soupape d'admission (3).

5. Dispositif selon une des revendications de 1 à 3,
**caractérisé en ce**
**qu'**avant la soupape d'admission (3), est raccordé un appareil de provocation à l'air froid.

6. Dispositif selon une des revendications de 1 à 5,
**caractérisé en ce**
**qu'**il est configuré de façon à être commandé par calculateur.

7. Dispositif selon une des revendications de 1 à 6,
**caractérisé en ce**
**qu'**un débitmètre (7) est disposé dans le canal d'écoulement (1).

8. Dispositif selon la revendication 6 ou 7,
**caractérisé en ce que**,
dans le canal d'écoulement (1), en amont du piège à refroidissement (5), est disposé un dispositif d'aspiration (11).

9. Dispositif selon la revendication 8,
**caractérisé en ce que**
le dispositif d'aspiration (11) est relié à une unité d'analyses (12).

10. Agent de diagnostic par inhalation destiné à déterminer l'état de santé des organes des voies respiratoires par l'air expiré, comportant des alcools supérieurs marqués par un isotope stable et pharmaceutiquement compatibles et éventuellement des ingrédients usuels en pharmacie.

11. Agent de diagnostic selon la revendication 10,
**caractérisé en ce que**
les alcools supérieurs sont des alcools au moins en C₈.

12. Agent de diagnostic selon la revendication 10 ou 11,
**caractérisé en ce que**
les alcools supérieurs sont marqués par du ¹³C.

13. Agent de diagnostic selon une des revendications de 10 à 12,
**caractérisé en ce**
**que** l'alcool supérieur marqué par un isotope stable est de l'hexadécan-1-ol marqué par du ¹³C.

14. Agent de diagnostic par inhalation destiné à déterminer l'état de santé des organes des voies respiratoires par l'air expiré, comportant des aminoacides marqués par un isotope stable et éventuellement des ingrédients pharmaceutiquement compatibles, dans lequel les aminoacides sont marqués par du ¹⁵N.

15. Agent de diagnostic selon la revendication 14,
**caractérisé en ce que**
l'aminoacide est de la L-arginine.

16. Utilisation d'alcools supérieurs marqués par un isotope stable et pharmaceutiquement compatibles ou d'aminoacides marqués par un isotope stable pour déterminer l'état de santé des organes des voies respiratoires par l'air expiré, au cours de laquelle ces composés sont inhalés.

17. Procédé de détermination de la capacité métabolique de l'épithélium des bronches,
**caractérisé en ce**
**qu'**au moyen du dispositif selon une des revendications de 1 à 9, des agents de diagnostic pouvant former des métabolites dans les organes des voies respiratoires sont ajoutés de manière dosée à l'air inspiré d'un sujet, et qu'ensuite on recueille les substances présentes dans l'air expiré et on analyse les métabolites et/ou la quantité restante d'agent de diagnostic.
